# FASCICULE DE BREVET EUROPEEN

(11) **EP 3 484 640 B1**
(45) Date de publication et mention de la délivrance du brevet: **12.08.2020**
(21) Numéro de dépôt: 17740117.1
(22) Date de dépôt: 11.07.2017
(51) Int. Cl.: B21D 28/34, A61B 17/00, A61B 17/16

(54) **PROCÉDÉ DE FABRICATION D'UN ALESOIR**
VERFAHREN ZUR HERSTELLUNG EINER REIBAHLE
METHOD FOR PRODUCING A REAMER

(30) Priorité: 12.07.2016 FR 1656688
(43) Date de publication de la demande: 22.05.2019
(73) Titulaire: Deuxventorio Sarl, 1196 Gland (CH)
(72) Inventeur: GRADEL, Thomas, 74970 Marignier (FR)
(74) Mandataire: Cabinet Poncet
(86) Numéro de dépôt international: PCT/IB2017/054172
(87) Numéro de publication internationale: WO 2018/011708

(56) Documents cités:
- EP-A2- 0 879 577
- US-A1- 2003 135 219
- US-A1- 2014 188 116

## Description

### DOMAINE TECHNIQUE DE L'INVENTION

La présente invention concerne un procédé de fabrication d'un alésoir, tel qu'une fraise destinée au fraisage de la cavité acétabulaire d'un patient lors d'une opération chirurgicale de la hanche.

On connaît un alésoir tel qu'une fraise destinée au fraisage de la cavité acétabulaire d'un patient, comprenant un corps de coupe creux sensiblement hémisphérique à paroi perforée. Pour former une dent sur une telle fraise, il est connu du document EP 0 879 577 A2 de procéder au moyen des trois étapes successives suivantes :
- lors d'une première étape, former un trou dans la paroi du corps de coupe pour définir les contours d'une languette,
- lors d'une deuxième étape, former de façon précise par fraisage une arête de référence et affûter précisément par fraisage une arête de coupe sur le bord libre de ladite languette ;
- lors d'une troisième étape, relever la languette avec un angle précis par enfermement de ladite languette entre un poinçon et une enclume, en provoquant un pliage dans une zone située à l'écart de l'arête de coupe

Former l'arête de coupe lors d'une étape préalable au relevage de la languette pour disposer l'arête de coupe en saillie par rapport à la paroi du corps de coupe rend le procédé de fabrication long et onéreux. De plus, une telle dent formée par une languette pliée a une faible rigidité structurelle et a tendance à se déformer lors des efforts appliqués par le chirurgien. Les caractéristiques de coupe d'une telle fraise ne sont donc pas fiables ni durables dans le temps.

Les documents US 2014/188116 A1, WO 2016/071867 A1, US 2006/095041 A1 et US 2003/135219 A1 décrivent quant à eux un procédé de fabrication comportant les étapes suivantes :
- percer la paroi du corps de coupe selon une direction de perçage sensiblement perpendiculaire à la paroi,
- chanfreiner par usinage (au moyen d'un foret ou d'une fraise) la périphérie du trou obtenu pour former une arête de coupe à profil aigu,
- déformer plastiquement par emboutissage une portion de paroi s'étendant radialement depuis et à l'écart du trou ménagé dans la paroi, pour former la dent.

L'ordre des étapes de réalisation du chanfrein par usinage et de déformation plastique par emboutissage est parfois inverse.

Lors d'un tel procédé, l'arête de coupe est fabriquée par usinage (retrait) d'une partie de la matière constitutive de la paroi en périphérie du trou, ce qui est long et onéreux. La face oblique du chanfrein procure une augmentation radiale progressive d'épaisseur en périphérie du trou, puis l'opération d'emboutissage permet de disposer une partie de ce chanfrein en saillie par rapport à la surface extérieure sensiblement hémisphérique du corps de coupe creux, pour obtenir une dent qui viendra tailler la matière osseuse lors du fraisage de la cavité acétabulaire d'un patient pendant une opération chirurgicale de la hanche.

Le chanfrein ne permet cependant pas toujours d'obtenir des capacités de coupe suffisantes, de sorte qu'il est souvent réalisé un affûtage manuel de la partie de chanfrein mise en saillie. Cet affûtage se fait dent par dent et prend beaucoup de temps. Le coût de fabrication exclut ainsi la possibilité de mettre en place une politique d'usage unique des alésoirs pour la réduction des risques infectieux au sein des établissements de santé.

En outre, il faut que l'opérateur en charge de l'affûtage ait une certaine dextérité pour obtenir des dents aux caractéristiques de coupe relativement uniformes. Si les dents n'ont pas des caractéristiques de coupe suffisamment uniformes à l'issue de leur affûtage, il peut se produire des efforts non uniformément répartis sur l'os lors du fraisage de la cavité acétabulaire, résultant en des vibrations pouvant conférer à la cavité acétabulaire une section transversale sensiblement polygonale (au lieu d'être circulaire). La bonne installation et ostéointégration de la cupule dans la cavité acétabulaire peut alors être compromise.

Enfin, les dents ont tendance à s'user trop rapidement. Ainsi, les caractéristiques de coupe des alésoirs diminuent assez (voire trop) vite dans le temps. Cela compromet la possibilité de réutiliser plusieurs fois un alésoir en vue de mieux en amortir les coûts d'acquisition.

Il doit être noté que, dans les documents US 2014/188116 A1, WO 2016/071867 A1, US 2006/095041 A1 et US 2003/135219 A1, il n'est guère fait usage que d'un poinçon. Il n'est nulle part explicité l'utilisation d'une enclume contre laquelle on viendrait effectuer un pressage.

Les documents US 2003/0181916 et US 2005/0113837 A1 décrivent un procédé de fabrication conforme au préambule de la revendication 1. Le bord du trou ne subit aucun affûtage préalable ou postérieur à l'emboutissage formant la dent en saillie sur la paroi du corps de coupe. Le procédé de fabrication des documents 2003/0181916 et US 2005/0113837 A1 est ainsi moins onéreux. Toutefois, la fiabilité et la durabilité dans le temps des caractéristiques de coupe des dents sont relativement insatisfaisantes. Les caractéristiques de coupe des dents sont en effet assez variables d'une dent à l'autre, et elles ont tendance à vite se détériorer dans le temps, notamment à l'issue de plusieurs utilisations de l'alésoir.

### EXPOSE DE L'INVENTION

Un problème proposé par la présente invention est de proposer un procédé de fabrication rapide et peu onéreux d'un alésoir, tel qu'une fraise destinée au fraisage de la cavité acétabulaire d'un patient.

Simultanément, la présente invention vise à fournir un procédé de fabrication facilement automatisable et permettant d'obtenir des caractéristiques de coupe plus fiables et plus durables dans le temps, même à l'issue de plusieurs utilisations.

Pour atteindre ces objets ainsi que d'autres, l'invention propose un procédé de fabrication d'un alésoir tel qu'une fraise destinée au fraisage de la cavité acétabulaire d'un patient et comprenant un corps de coupe creux sensiblement hémisphérique à paroi perforée, ledit procédé comprenant une étape lors de laquelle on forme au moins une dent par emboutissage de la paroi au moyen d'un poinçon venant déformer plastiquement une portion de paroi s'étendant radialement depuis et à l'écart d'un trou ménagé dans la paroi ; selon l'invention, lors de l'emboutissage de la dent, une zone de la portion de paroi déformée, adjacente au trou, est amincie et pressée par le poinçon contre une enclume.

L'emboutissage et le pressage subséquent entre le poinçon et l'enclume de la zone de la portion de paroi déformée procurent un amincissement de la paroi étirée pour former la dent en saillie sur la paroi du corps de coupe. Cet amincissement procure des capacités de coupe au bord libre de la dent ainsi formée. En maîtrisant convenablement l'effort de pressage, on parvient à un amincissement mieux maîtrisé et reproductible pour des caractéristiques de coupe plus uniformes entre toutes les dents.

Cet amincissement ne s'effectue pas par retrait de la matière mais par étirement, puis mise sous contraintes et fluage lors d'un pincement. L'arête de coupe est ainsi formée lors de l'emboutissage. Lors du pressage (ou pincement), il se produit alors une sorte d'écrouissage local de la dent (ou un fibrage de la matière) qui lui confère une résistance structurelle accrue et une meilleure durabilité dans le temps au cours de la réutilisation de l'alésoir.

La formation de la dent est facilement automatisable, rapide, et peut être mise en œuvre aisément dans un outil à suivre par exemple. Le coût de fabrication de l'alésoir est ainsi très fortement diminué.

De préférence, on peut prévoir que :
- le poinçon comporte une extrémité libre à surface de pressage réglée,
- lors de l'étape de formation de la dent, la génératrice de la surface de pressage est oblique par rapport au plan défini par l'orifice du trou.

Un tel poinçon est relativement simple et peu onéreux à fabriquer.

De préférence, on peut prévoir que :
- l'enclume comporte une surface de pressage réglée dont la génératrice est oblique par rapport au plan défini par l'orifice du trou,
- lors de la formation de la dent, l'angle entre la génératrice de la surface de pressage de l'enclume et le plan défini par l'orifice du trou est inférieur à l'angle entre la génératrice de la surface de pressage du poinçon et le plan défini par l'orifice du trou.

Cette différence d'angle permet d'obtenir un bon amincissement progressif avec un fort écrouissage ou fibrage de la matière au voisinage du bord libre de la dent (destiné à constituer l'arête de coupe de la dent). On conserve en revanche une épaisseur de matière plus importante en se déplaçant à l'écart de l'arête de coupe et du trou, ce qui procure une bonne rigidité structurelle de la dent. En pratique, la différence d'angle peut de préférence être d'environ 2 degrés.

Avantageusement, on peut prévoir que, jusqu'à l'étape de formation de la dent, le corps de coupe se présente sous la forme d'un flan métallique plat perforé. On peut ainsi former plusieurs dents simultanément par l'usage de plusieurs poinçons parallèles entre eux.

Avantageusement, le flan métallique plat peut présenter une épaisseur comprise entre environ 0,4 mm et environ 1 mm.

De préférence, après formation de la dent, le flan plat peut être découpé pour obtenir une pluralité de pétales perforés et dentés s'étendant radialement depuis une zone centrale depuis laquelle les pétales s'étendent jusqu'à une extrémité libre, et séparés les uns des autres par des espaces latéraux radiaux.

Avantageusement, après découpage du flan plat pour former les pétales, on peut :
- conformer le flan plat en demi-sphère,
- rapporter et fixer les extrémités libres des pétales sur un corps de base au moins partiellement circulaire.

Avantageusement, le corps de coupe peut être en acier inoxydable, de préférence en acier inoxydable de nuance 304L ou 316L.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue en perspective d'un exemple de mode de réalisation d'alésoir selon l'invention ;
- la figure 2 est une vue en perspective d'un poste de travail d'un outil à suivre ;
- la figure 3 est une vue de face d'un poinçon ;
- la figure 4 est une vue de côté du poinçon de la figure 3 ;
- la figure 5 est une vue en perspective du poinçon de la figure 3 ;
- les figures 6 à 8 sont des vues en perspective et en coupe du deuxième poste de travail sur lequel est embouti un flan métallique plat pour former une dent ;
- la figure 9 est une vue de détail et de côté de la figure 8 ;
- la figure 10 est une vue en perspective et en coupe du poste de travail de la figure 2 après emboutissage du flan métallique pour former une dent ;
- la figure 11 est une vue en perspective du flan métallique après emboutissage pour former une dent ;
- la figure 12 est une vue schématique et en perspective illustrant un procédé de fabrication de l'alésoir de la figure 1.

### DESCRIPTION DES MODES DE REALISATION PREFERES

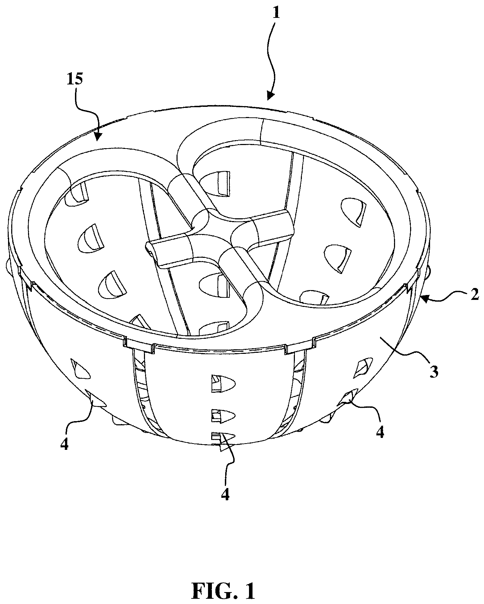

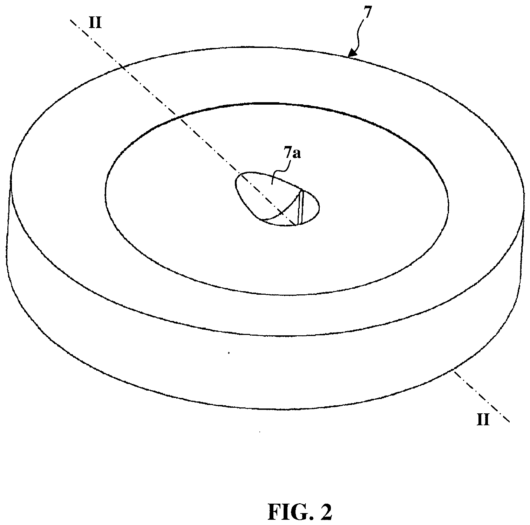

Sur la figure 1 est illustré un alésoir 1 qui est une fraise destinée au fraisage de la cavité acétabulaire d'un patient. L'alésoir 1 comprend un corps de coupe 2 creux sensiblement hémisphérique à paroi 3 perforée. Une pluralité de dents 4 ont été formées par déformation de la paroi 3. Leur formation est plus particulièrement réalisée par emboutissage dans un outil à suivre, comme il sera expliqué ci-après à l'aide des figures 2 à 12.

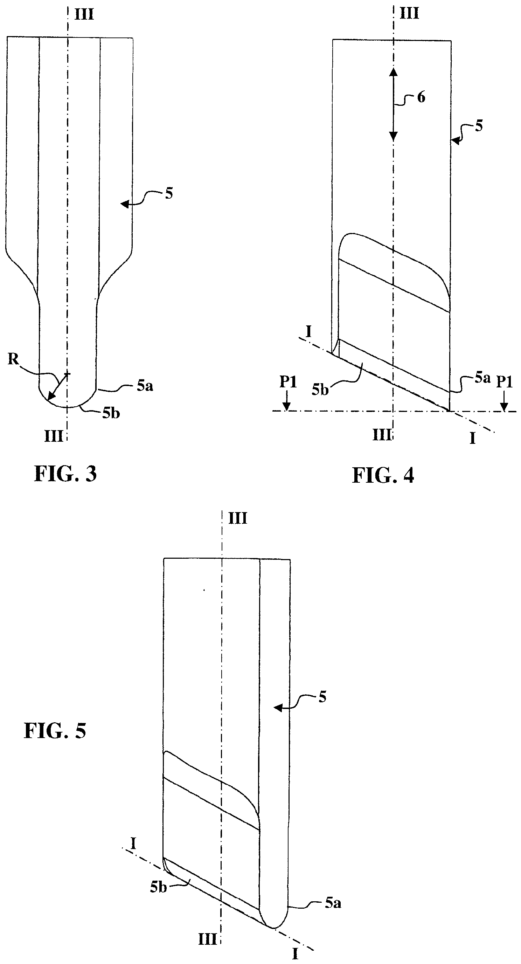

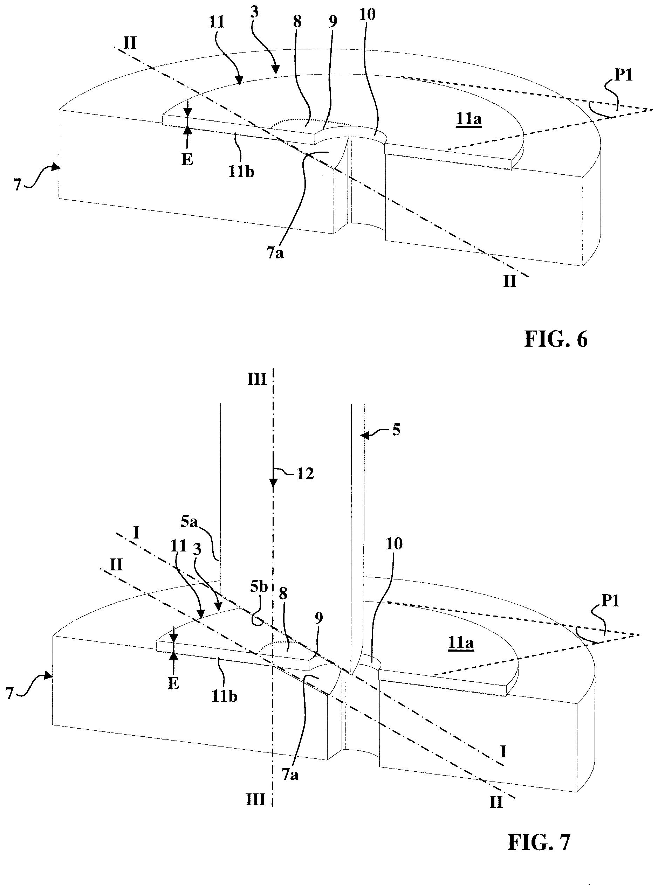

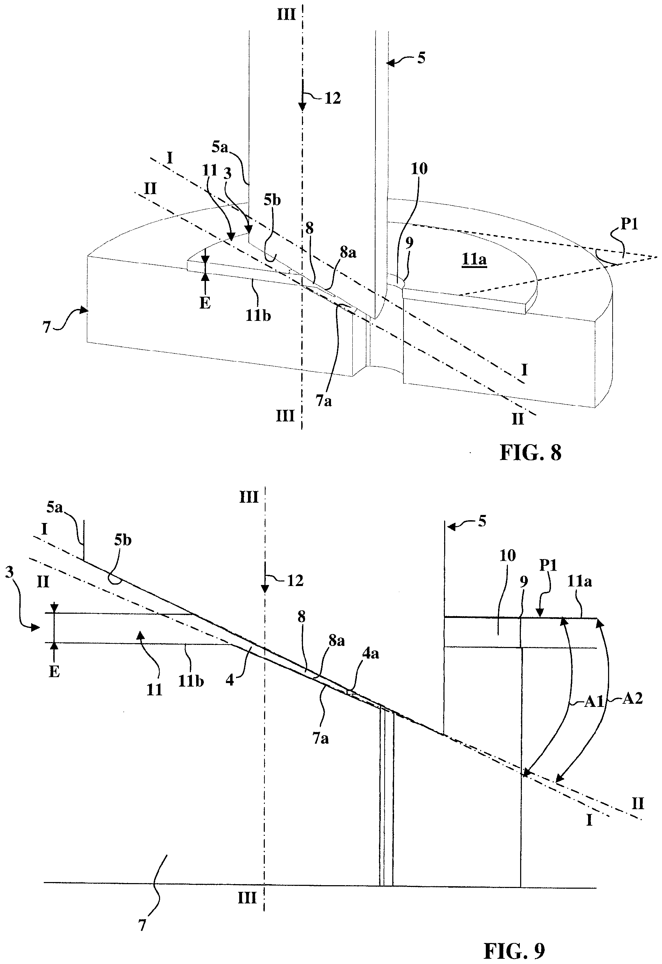

Lors dudit emboutissage, il est fait usage d'un poinçon 5 tel qu'illustré sur les figures 3 à 5. Le poinçon 5 comporte une extrémité libre 5a à surface de pressage réglée 5b dont la section transversale est en arc de cercle de rayon R (figure 3). Le poinçon 5 est destiné à être animé d'un mouvement de translation bidirectionnelle selon la direction axiale III-III illustrée par la double flèche 6 tandis qu'une paroi 3 à déformer se trouve dans le plan P1 illustré sur la figure 6. Ainsi, lors de l'étape de formation d'une dent 4, la génératrice I-I de la surface de pressage 5b est oblique par rapport au plan P1 (figure 9).

Lors dudit emboutissage, il est simultanément fait usage d'une enclume 7 telle qu'illustrée sur la figure 2. L'enclume 7 comporte une surface de pressage 7a réglée ayant également une section transversale en arc de cercle de rayon légèrement supérieur au rayon R. Comme il est plus particulièrement visible sur la figure 6, la génératrice II-II de la surface de pressage 7a de l'enclume 7 est oblique par rapport au plan P1 dans lequel se trouve la face de la paroi 3 qui n'est pas au contact de l'enclume 7. De façon plus spécifique, le plan P1 est défini par l'orifice 9 du trou 10 ménagé dans la paroi 3.

Les surfaces de pressage 5b et 7a peuvent avoir une section transversale de forme différente. On peut par exemple envisager une section transversale constituée par la succession de trois arcs de cercle de rayons différents, notamment avec les premier et troisième rayons égaux et inférieurs au deuxième rayon. La surface de pressage 5b a alors un sommet (tandis que la surface de pressage 7a a un fond) un peu aplati.

Sur la vue de détail de la figure 9 illustrant plus précisément la coopération entre le poinçon 5 et l'enclume 7 lors de la formation d'une dent 4, on voit que l'angle A2 entre la génératrice II-II de la surface de pressage 7a de l'enclume 7 et le plan P1 est inférieure à l'angle A1 entre la génératrice I-I de la surface de pressage 5b du poinçon 5 et le plan P1. Cette différence est d'environ 2 degrés.

Lors de la fabrication de l'alésoir 1 de la figure 1, jusqu'à l'étape de formation de la dent 4, le corps de coupe 2 se présente sous la forme d'un flan 11 métallique plat partiellement illustré sur la figure 6. Ce flan 11 métallique plat est pourvu d'un trou 10 à orifice 9. Ce trou 10 est de préférence réalisé sur un poste de travail précédent de l'outil à suivre, et sur lequel le flan 11 est découpé par poinçonnage. Le trou 10 définit le plan P1, qui coïncide ici avec le plan de la face supérieure 11a du flan 11 métallique plat.

Le flan 11 métallique plat présente une épaisseur E comprise entre environ 0,4 mm et environ 1 mm. Le flan 11 métallique plat est en acier inoxydable de nuance 304L ou 316L.

Sur la figure 6, le flan 11 repose en appui sur l'enclume 7 selon une face 11b. Une portion 8 de paroi 3 s'étend radialement depuis et à l'écart du trou 9 ménagé dans la paroi 3, et est située en correspondance de la surface de pressage 7a de l'enclume 7.

Lors d'une étape ultérieure, illustrée sur la figure 7, le poinçon 5 est déplacé en direction de l'enclume 7 (tel qu'illustré par la flèche 12) selon une direction axiale III-III sensiblement perpendiculaire au plan P1.

Lors de ce déplacement, le poinçon 5 vient déformer plastiquement la portion 8 de paroi 3 comme illustré sur la figure 8. Par principe de conservation de la matière, une zone 8a de la portion 8 de paroi 3 déformée, adjacente au trou 10, subi un amincissement du fait de son déplacement en saillie par rapport à la face 11b du flan plat 11.

Au bout d'une certaine course du poinçon 5, la zone 8a de la portion 8 de paroi 3 déformée, adjacente au trou 10, est pressée par le poinçon 5 contre l'enclume 7 (figure 9).

L'emboutissage et le pressage subséquent entre le poinçon 5 et l'enclume 7 de la zone 8a de la portion 8 de paroi 3 déformée procurent un étirement et un amincissement de la paroi 3 pour former la dent 4. Cet amincissement procure des capacités de coupe au bord libre 4a de la dent 4 ainsi formée. En maîtrisant convenablement l'effort de pressage, on parvient à un amincissement bien maîtrisé et répétable pour des caractéristiques de coupe sensiblement identiques pour toutes les dents 4.

Le pincement entre le poinçon 5 et l'enclume 7 procure une sorte d'écrouissage local de la dent 4 (ou un fibrage de la matière) qui lui confère une résistance structurelle accrue et une meilleure durabilité dans le temps au cours de l'utilisation de l'alésoir 1.

Ici, la totalité de la portion 8 de paroi 3 se trouve pincée entre la surface de pressage 5b du poinçon 5 et la surface de pressage 7a de l'enclume 7. On pourrait toutefois ne pincer qu'une partie de la portion 8, ladite partie s'étendant à partir du trou 10 pour au moins former le bord libre 4a de la dent 4 en lui conférant des propriétés de coupe et une résistance structurelle satisfaisantes.

On observe sur la figure 9 que, du fait de la différence entre les angles A1 et A2, l'épaisseur de la portion 8 constituant la dent 4 augmente progressivement à l'écart du bord libre 4a de la dent 4.

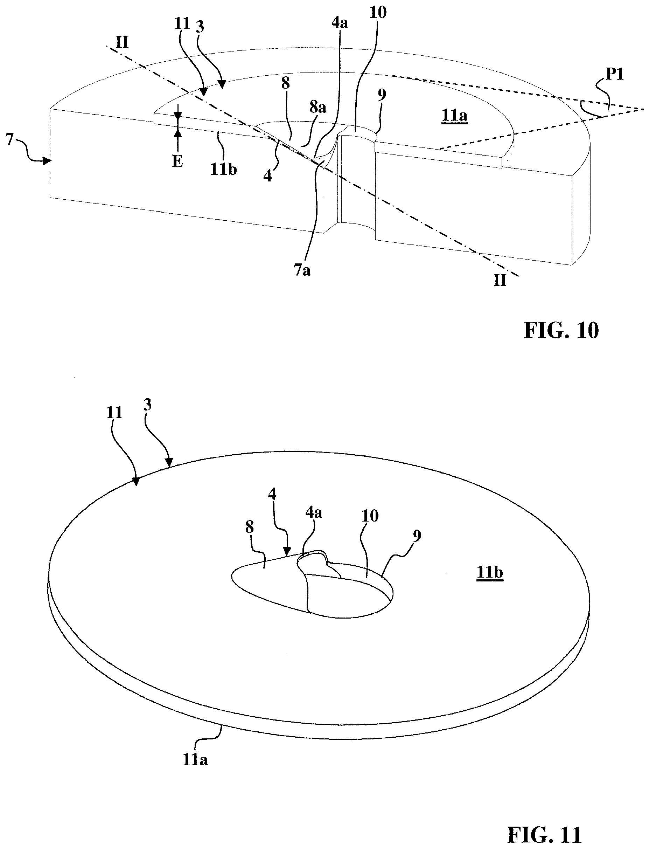

Le poinçon 5 est ensuite déplacé à l'écart de l'enclume 7 selon un mouvement inverse de celui illustré par la flèche 12 sur la figure 7. Le flan 11 se trouve alors dans la configuration illustrée sur la figure 10, et est ensuite retiré de l'enclume 7 au moyen d'éjecteurs.

Sur la figure 11 est partiellement illustré le flan 11 muni de la dent 4 qui forme une saillie sur la face 11b.

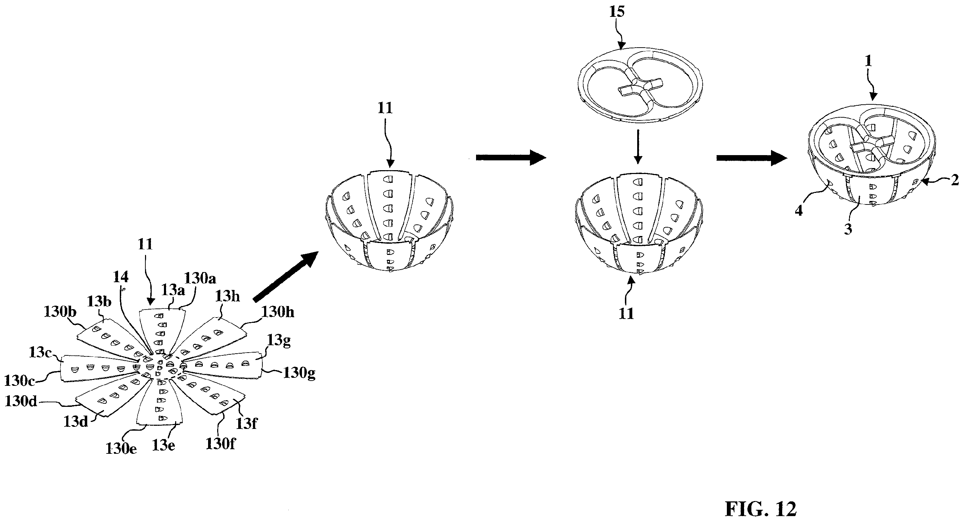

Une fois que toutes les dents 4 sont formées dans le flan 11 métallique plat, ce dernier est découpé pour obtenir une pluralité de pétales 13a à 13h perforés et dentés s'étendant radialement depuis une zone centrale 14 depuis laquelle les pétales 13a à 13h s'étendent jusqu'à une extrémité libre 130a à 130h. Les pétales 13a à 13h sont séparés les uns des autres par des espaces latéraux radiaux (étape a) sur la figure 12).

Ensuite, lors d'une étape b), on conforme le flan 11 plat en demi-sphère. Puis, lors d'une étape c), on rapporte et on fixe les extrémités libres 130a à 130h des pétales 13a à 13h sur un corps de base 15 au moins partiellement circulaire. Le flan 11 est alors maintenu en forme de dôme sensiblement hémisphérique pour former un corps de coupe 2 apte au fraisage de la cavité acétabulaire d'un patient.

La présente invention n'est pas limitée aux modes de réalisation qui ont été explicitement décrits, mais elle en inclut les diverses variantes et généralisations contenues dans le domaine des revendications ci-après.

## Revendications

1. Procédé de fabrication d'un alésoir (1), tel qu'une fraise destinée au fraisage de la cavité acétabulaire d'un patient, l'alésoir comprenant un corps de coupe (2) creux sensiblement hémisphérique à paroi (3) perforée, ledit procédé comprenant une étape lors de laquelle on forme au moins une dent (4) par emboutissage de la paroi (3) au moyen d'un poinçon (5) venant déformer plastiquement une portion (8) de paroi (3) s'étendant radialement depuis et à l'écart d'un trou (10) ménagé dans la paroi (3), **caractérisé en ce que**, lors de l'emboutissage de la dent (4), une zone (8a) de la portion (8) de paroi (3) déformée, adjacente au trou (10), est amincie et pressée par le poinçon (5) contre une enclume (7).

2. Procédé de fabrication selon la revendication 1, **caractérisé en ce que** :
- le poinçon (5) comporte une extrémité libre (5a) à surface de pressage (5b) réglée,
- lors de l'étape de formation de la dent (4), la génératrice (I-I) de la surface de pressage (5b) est oblique par rapport au plan (P1) défini par l'orifice (9) du trou (10).

3. Procédé de fabrication selon la revendication 2, **caractérisé en ce que** :
- l'enclume (7) comporte une surface de pressage (7a) réglée dont la génératrice (II-II) est oblique par rapport au plan (P1) défini par l'orifice (9) du trou (10),
- lors de la formation de la dent (4), l'angle (A2) entre la génératrice (II-II) de la surface de pressage (7a) de l'enclume (7) et le plan (P1) défini par l'orifice (9) du trou (10) est inférieur à l'angle (A1) entre la génératrice (I-I) de la surface de pressage (5b) du poinçon (5) et le plan (P1) défini par l'orifice (9) du trou (10), de préférence d'environ 2 degrés.

4. Procédé de fabrication selon l'une quelconque des revendications 1 à 3, **caractérisé en ce que**, jusqu'à l'étape de formation de la dent (4), le corps de coupe (2) se présente sous la forme d'un flan (11) métallique plat perforé.

5. Procédé de fabrication selon la revendication 4, **caractérisé en ce que** le flan (11) métallique plat présente une épaisseur (E) comprise entre 0,4 mm et 1 mm.

6. Procédé de fabrication selon l'une des revendications 4 ou 5, **caractérisé en ce que**, après formation de la dent (4), le flan (11) plat est découpé pour obtenir une pluralité de pétales (13a-13h) perforés et dentés s'étendant radialement depuis une zone centrale (14) depuis laquelle les pétales (13a-13h) s'étendent jusqu'à une extrémité libre (130a-130h), et séparés les uns des autres par des espaces latéraux radiaux.

7. Procédé de fabrication selon la revendication 6, **caractérisé en ce que**, après découpage du flan (11) plat pour former les pétales (13a-13h) :
- on conforme le flan (11) plat en demi-sphère,
- on rapporte et fixe les extrémités libres (130a-130h) des pétales (13a-13h) sur un corps de base (15) au moins partiellement circulaire.

8. Procédé de fabrication selon l'une quelconque des revendications 1 à 7, **caractérisé en ce que** le corps de coupe (2) est en acier inoxydable, de préférence en acier inoxydable de nuance 304L ou 316L.

## Patentansprüche

1. Verfahren zur Herstellung einer Reibahle (1), wie einer Fräse, vorgesehen zum Fräsen der Hüftgelenkspfanne eines Patienten, die Reibahle umfassend einen hohlen, im Wesentlichen halbkugelförmigen, Schneidkörper (2) mit einer perforierten Wandung (3), wobei besagtes Verfahren einen Schritt umfasst, während dessen mindestens ein Zahn (4) durch Umformen der Wandung (3) mittels eines Stempels (5) gebildet wird, um dabei einen sich radial erstreckenden Abschnitt (8) der Wandung (3) von und weg von einem Loch (10), das in der Wandung (3) ausgebildet ist, plastisch zu verformen, **dadurch gekennzeichnet, dass** während des Stanzens des Zahns (4) eine Zone (8a) des Abschnitts (8) der verformten Wandung (3) neben dem Loch (10) gedünnt und durch den Stempel (5) gegen einen Amboss (7) gepresst wird.

2. Herstellungsverfahren nach Anspruch 1, **dadurch gekennzeichnet, dass**:
• der Stempel (5) weist ein freies Ende (5a) mit geregelter Pressfläche (5b) auf,
• während des Schritts der Bildung des Zahns (4) ist der Erzeuger (I-I) der Pressfläche (5b) schräg zu der Ebene (P1), die durch die Öffnung (9) des Lochs (10) definiert ist.

3. Herstellungsverfahren nach Anspruch 2, **dadurch gekennzeichnet, dass**:
• der Amboss (7) weist eine geregelte Pressfläche (7a) auf, deren Erzeuger (II-II) schräg ist zu der Ebene (P1), die durch die Öffnung (9) des Lochs (10) definiert ist,
• während der Bildung des Zahns (4) ist der Winkel (A2) zwischen dem Erzeuger (II-II) der Pressfläche (7a) des Ambosses (7) und der durch die Öffnung (9) des Lochs (10) definierten Ebene (P1) kleiner als der Winkel (A1) zwischen dem Erzeuger (I-I) der Pressfläche (5b) des Stempels (5) und der durch die Öffnung (9) des Lochs (10) definierten Ebene (P1), vorzugsweise etwa 2 Grad.

4. Herstellungsverfahren nach einem der Ansprüche 1 bis 3, **dadurch gekennzeichnet, dass** bis zu dem Schritt der Bildung des Zahns (4) der Schneidkörper (2) in einer Form als flacher perforierter Metallrohling (11) vorliegt.

5. Herstellungsverfahren nach Anspruch 4, **dadurch gekennzeichnet, dass** der flache Metallrohling (11) mit einer Dicke (E), umfassend zwischen 0,4 mm und 1 mm, vorliegt.

6. Herstellungsverfahren nach einem der Ansprüche 4 oder 5, das **dadurch gekennzeichnet ist, dass** nach der Bildung des Zahns (4) der flache Rohling (11) geschnitten wird, um mehrere perforierte und mit Zähnen versehene Blütenblätter (13a-13h) zu erhalten, die sich radial von einer zentralen Zone (14) erstrecken, von der sich die Blütenblätter (13a-13h) bis zu einem freien Ende (130a-130h) erstrecken und durch radiale seitliche Zwischenräume voneinander getrennt sind.

7. Herstellungsverfahren nach Anspruch 6, **dadurch gekennzeichnet, dass** nach dem Schneiden des flachen Rohlings (11), um die Blütenblätter (13a-13h) zu bilden:
• Anpassen des flachen Rohlings (11) an die Halbkugel,
• Befestigen und Fixieren der freien Enden (130a-130h) der Blütenblätter (13a-13h) an einem zumindest teilweise kreisförmigen Grundkörper (15).

8. Herstellungsverfahren nach einem der Ansprüche 1 bis 7, **dadurch gekennzeichnet, dass** der Schneidkörper (2) ein rostfreier Stahl ist, vorzugsweise rostfreier Stahl der Klasse 304L oder 316L.

## Claims

1. A method for producing a reamer (1), such as a milling cutter intended to mill the acetabular cavity of a patient, the reamer comprising a substantially hemispherical hollow cutter body (2) with perforated wall (3), said method comprising a step in which at least one tooth (4) is formed by stamping the wall (3) by means of a punch (5) plastically deforming a portion (8) of wall (3) extending radially from and away from a hole (10) formed in the wall (3), **characterized in that**, in the stamping of the tooth (4), a zone (8a) of the deformed portion (8) of wall (3), adjacent to the hole (10), is thinned and pressed by the punch (5) against an anvil (7).

2. The production method as claimed in claim 1, **characterized in that**:
- the punch (5) comprises a free end (5a) with set pressing surface (5b),
- in the step of forming of the tooth (4), the generatrix (I-I) of the pressing surface (5b) is oblique relative to the plane (P1) defined by the orifice (9) of the hole (10).

3. The production method as claimed in claim 2, **characterized in that**:
- the anvil (7) comprises a set pressing surface (7a) whose generatrix (II-II) is oblique relative to the plane (P1) defined by the orifice (9) of the hole (10),
- in the forming of the tooth (4), the angle (A2) between the generatrix (II-II) of the pressing surface (7a) of the anvil (7) and the plane (P1) defined by the orifice (9) of the hole (10) is smaller than the angle (A1) between the generatrix (I-I) of the pressing surface (5b) of the punch (5) and the plane (P1) defined by the orifice (9) of the hole (10), preferably by approximately 2 degrees.

4. The production method as claimed in any one of claims 1 to 3, **characterized in that**, until the step of forming of the tooth (4), the cutter body (2) takes the form of a perforated flat metal blank (11).

5. The production method as claimed in claim 4, **characterized in that** the flat metal blank (11) has a thickness (E) of between 0.4 mm and 1 mm.

6. The production method as claimed in one of claims 4 and 5, **characterized in that**, after the forming of the tooth (4), the flat blank (11) is cut to obtain a plurality of perforated and toothed petals (13a-13h) extending radially from a central zone (14) from which the petals (13a-13h) extend to a free end (130a-130h), and separated from one another by radial lateral spaces.

7. The production method as claimed in claim 6, **characterized in that**, after the cutting of the flat blank (11) to form the petals (13a-13h):
- the flat blank (11) is shaped as a hemisphere,
- the free ends (130a-130h) of the petals (13a-13h) are added and fixed onto an at least partially circular base body (15).

8. The production method as claimed in any one of claims 1 to 7, **characterized in that** the cutter body (2) is made of stainless steel, preferably of 304L or 316L grade stainless steel.
